(19) **Europäisches Patentamt**
**European Patent Office**
**Office européen des brevets**

(11) Veröffentlichungsnummer: **0 275 543 B1**

(12) **EUROPÄISCHE PATENTSCHRIFT**

(45) Veröffentlichungstag der Patentschrift: **11.12.91**

(51) Int. Cl.⁵: **A61F 5/01**

(21) Anmeldenummer: **87119181.3**

(22) Anmeldetag: **24.12.87**

(54) **Stütze für den Sprunggelenkbereich.**

(30) Priorität: **05.01.87 DE 8700201 U**

(43) Veröffentlichungstag der Anmeldung:
**27.07.88 Patentblatt 88/30**

(45) Bekanntmachung des Hinweises auf die
Patenterteilung:
**11.12.91 Patentblatt 91/50**

(84) Benannte Vertragsstaaten:
**AT BE CH DE FR GB IT LI NL SE**

(56) Entgegenhaltungen:
**DE-U- 8 617 783**
**GB-A- 2 167 964**
**US-A- 3 527 211**
**US-A- 4 280 488**

(73) Patentinhaber: **Rau, Roland, Dr. med.**
**Hauptstrasse 8**
**W-7614 Gengenbach(DE)**

(72) Erfinder: **Rau, Roland, Dr. med.**
**Hauptstrasse 8**
**W-7614 Gengenbach(DE)**

(74) Vertreter: **Becker, Maria, Dipl.-Phys.**
**Auf dem Haigst 29 Postfach 70 01 47**
**W-7000 Stuttgart 70(DE)**

## Beschreibung

Die Erfindung betrifft eine Stütze für den Sprunggelenkbereich mit einem sowohl den Fusswurzelbereich als auch den Fersenbereich sowie einen Teil des Unterschenkels zumindest teilweise umschliessenden und in Stützrichtung weitgehend starren Stützschaft, der vorderseitig eine Öffnung aufweist, die mittels verstellbarer, vorzugsweise Klettverschlüsse aufweisen der Verschlusselemente verschliessbar ist. Eine solche Stütze ist aus der GB-A-2.167.964 bekannt. Diese Stütze dient der Fusskorrektur bei Kindern zur Vorfussadduktion.

Es ist ferner bereits bekannt, bei Verletzungen im Sprunggelenkbereich eine Gelenkmanschette vorzusehen, um eine seitliche Abstützung und damit eine Ruhigstellung des unteren Sprunggelenkes zu erzielen. Diese bekannte Gelenkmanschette ist steigbügelartig bzw. U-förmig ausgebildet, wobei die seitlich am Unterschenkel anliegenden Enden mit einer Bandage fixiert werden müssen. Die auftretenden Stützkräfte, die in entgegengesetzte Schiebekräfte in den Manschettenenden umgesetzt werden, müssen hierbei von der Bandage aufgenommen werden. Dementsprechend muss diese vergleichsweise fest gewickelt sein, was aber einer guten Blutzirkulation abträglich und auch unbequem ist. Ausserdem ist dadurch das Anlegen der Gelenkmanschette umständlich und erfordert auch erhebliche Sachkunde.

Aufgabe der vorliegenden Erfindung ist es, eine Stütze der eingangs genannten Art zu schaffen, die eine verbesserte Seiten-Stützstabilität aufweist, gleichzeitig einfach anzulegen und bequem zu tragen ist.

Zur Lösung dieser Aufgabe wird erfindungsgemäss vorgeschlagen, dass sich der Stützschaft einerseits etwa über die Fusswurzelknochen bzw. etwa die halbe Fusslänge und andererseits bis wenigstens etwa 5 cm oberhalb der Knöchel erstreckt und im Bereich der Knöchel Ausnehmungen aufweist, die durch eine Aussenhaut überdeckt sind. Diese Stütze bildet einen stiefelartigen Teilschuh, wobei die Seitenteile insbesondere über die rückseitige Verbindung gegeneinander eine gute Stabilisierung erhalten, was einem Umknicken des Fusses entgegenwirkt. Andererseits ist dadurch noch ein bequemes Gehen und Abrollen des Fusses möglich. Eine Bandage ist dadurch nicht mehr erforderlich. die Stütze lässt sich somit auch von Laien problemlos anlegen.

Durch die Ausnehmungen im Bereich der Knöchel und vorzugsweise auch der Ferse werden einerseits Druckstellen in diesen empfindlichen Bereichen vermieden, andererseits wird durch die darüber gespannte Aussenhaut eine weiche Abstützung des Gewebes in diesem Bereich erreicht, so dass der Bildung von sogenannten Fensterödemen entgegengewirkt wird.

Diese Wirkung wird noch erhöht, wenn gemäss einer vorteilhafter Ausführungsform der Teilschuh innenseitig, gegebenenfalls ganzflächig, eine elastisch nachgiebige Auflage (Innenhaut), insbesondere Schaumstoff, aufweist.

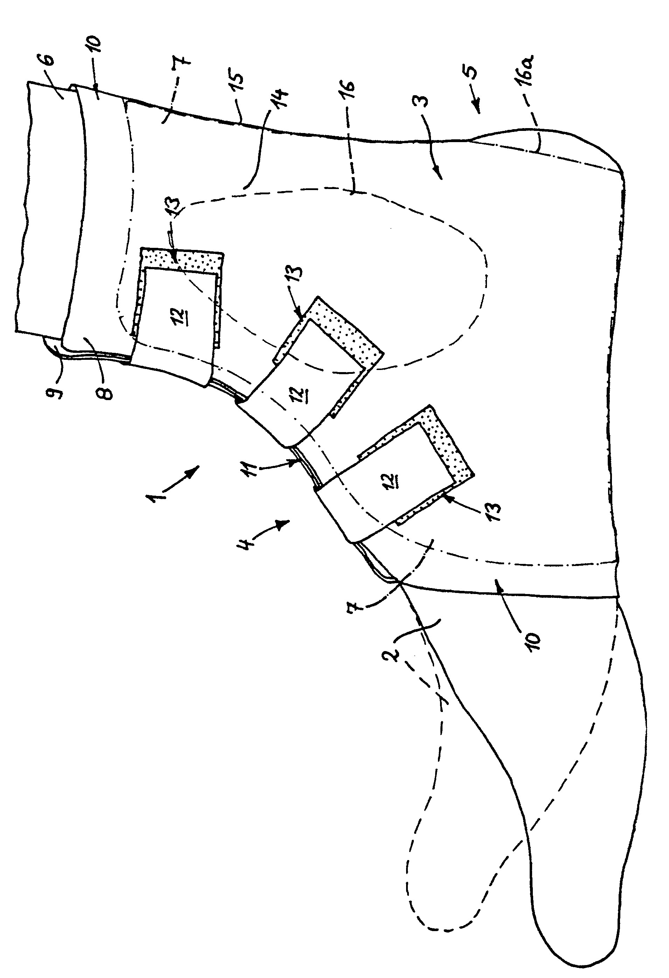

Zusätzliche Ausgestaltungen der Erfindung sind in den weiteren Unteransprüchen aufgeführt. Nachstehend ist die Erfindung mit ihren wesentlichen Einzelheiten anhand der Zeichnung noch erläutert.

Die einzige Figur zeigt:
eine Seitenanischt einer erfindungsgemässen Stütze in Funktionslage.

Eine in der Figur gezeigte Stütze 1 dient zur seitlichen Abstützung des unteren Sprunggelenkes eines menschlichen Fusses 2. Die erfindungsgemässe Stütze 1 ist als Teilschuh 3 ausgebildet, der etwa die vordere Hälfte des Fusses freilässt und den Fusswurzelbereich 4, den Fersenbereich 5 sowie einen Teil des Unterschenkels 6 umschliesst. Dadurch ist in Seitenrichtung eine gute Abstützung gegeben, andererseits besteht aber noch die Möglichkeit, mit angelegter Stütze 1 gut laufen zu können. Die für das Laufen notwendige Bewegbarkeit des Fusses ist strichliniert im vorderen Fussbereich angedeutet.

Der Teilschuh 3 weist einen aus weitgehend starrem Material, vorzugsweise aus Kunststoff, bestehenden Stützschaft 7 auf, dessen Aussenrandverlauf innerhalb des Teilschuhes 3 strichpunktiert umgrenzt ist. Dieser Stützschaft 7 ist durch eine Aussenhaut 8 und eine Innenhaut 9, z.B. aus dünnem Kunstleder, überdeckt, wobei diese Beschichtungen den Stützschaft 7 an seinen Rändern überragen. In diesem Übersand 10 sind die Aussenhaut 8 und die Innenhaut 9 miteinander verbunden, insbesondere miteinander verklebt. Der Teilschuh ist vorderseitig öffenbar ausgebildet und weist in diesem Trennbereich 11 durch Laschen 12 mit Klettverschlüssen 13 gebildete Verschlusselemente auf. Im Trennbereich 11 lässt sich nach dem Öffnen der Klettverschlüsse der Teilschuh 3 etwa elastisch auffedern, so dass ein bequemes Anlegen bzw. auch Ausziehen des Teilschuhes 3 möglich ist.Trotz dieser elastischen Auseinanderfederbarkeit des Teilschuhes 3 ergibt sich in seitlicher Stützrichtung eine gute Stabilität, da die Seitenteile 14 des Stützschaftes 7 rückseitig einstückig miteinander verbunden sind. Die Kraftübertragung bei einer Seitenbelastung der Stütze 1 wird somit über diese Verbindung 15 übertragen, wobei sich die Seitenteile 14 entsprechend gegenseitig stabilisieren. Prinzipiell übernimmt somit diese rückseitige Verbindung 15 die Funktion einer sonst bei einer bekannten Gelenkmanschette notwendigen Wickelbinde. Diese ist somit bei der erfindungsgemässen Stütze 1 nicht erforderlich, so dass die Handhabung beim Anlegen und Ausziehen der Stütze 1 wesentlich vereinfacht ist. Die Bedienung beim Anziehen und Ausziehen der Stütze beschränkt sich somit auf das einfache Öffnen und Schliessen der Klettverschlüsse 13.

Der erfindungsgemässe Teilschuh 3 ist vorzugsweise dünnwandig ausgebildet, wodurch er wenig aufträgt, so dass er als Innenschuh getragen werden kann. Auch dies ist erst möglich durch die gute Stabilisierung der Seitenteile 14 durch die rückseitige Verbindung 15.

Für einen Langzeittragekomfort ist es noch von wesentlicher Bedeutung, dass im Bereich der Knöchel und auch im Fersenbereich Ausnehmungen 16, 16a im Stützschaft 7 vorgesehen sind. In diesen vorstehenden Fussbereichen wird dadurch eine unerwünschte Druckbelastung vermieden. Die Innenhaut und auch die Aussenhaut sind über diese Bereiche hinweggespannt, so dass zwar keine Beaufschlagung durch den vergleichsweise harte Stützschaft 7 vorhanden ist, jedoch noch eine Abstützung, durch die insbesondere auch sogenannte Fensterödeme verhindert werden können.

Wie in der Zeichnung gut ersichtlich, erstreckt sich die Stütze 1 bzw. deren Stützschaft 7 etwa über die halbe Länge des Fusses, so dass der Vorderfussbereich noch gut zum Laufen bewegbar ist. Nach oben erstreckt sich der Stützschaft 7 stiefelschaftartig über den Knöchelbereich nach oben hinaus, wobei die Länge des über den Knöchelbereich überstehenden Schaftbereiches beispielsweise etwa 5 cm betragen kann. Es ergibt sich dann eine Gesamthöhe der Stütze 1 von der Fusssohle bis ans obere Ende von etwa 12 bis 20 cm.

Die erfindungsgemässe Stütze 1 kann in unterschiedlichen Grössen, vorzugsweise für bestimmte Fussgrössenbereiche vorgefertigt sein, so dass keine Anpassarbeiten an der Stütze 1 mehr erforderliche sind.

## Patentansprüche

1. Stütze für den Sprunggelenkbereich mit einem sowohl den Fusswurzelbereich (4) als auch den Fersenbereich (5) sowie einen Teil des Unterschenkels (6) zumindest teilweise umschliessenden und in Stützrichtung weitgehend starren Stützschaft (7), der vorderseitig eine Öffnung (11) aufweist, die mittels verstellbarer, vorzugsweise Klettverschlüsse aufweisender Verschlusselemente verschliessbar ist,

   **dadurch gekennzeichnet**

   dass sich der Stützschaft (7) einerseits etwa über die Fusswurzelknochen (4) bzw. etwa die halbe Fusslänge und andererseits bis wenigstens etwa 5 cm oberhalb der Knöchel erstreckt und im Bereich der Knöchel Aufnehmungen (16) aufweist, die durch eine Aussenhaut (8) überdeckt sind.

2. Stütze nach Anspruch 1, dadurch gekennzeichnet, dass der Stützschaft (7) auch im Bereich der Ferse eine Ausnehmung (16a) aufweist.

3. Stütze nach einem der vorhergehenden Ansprüche, dadurch gekennzeichnet, dass der Teilschuh (3) innenseitig, gegebenenfalls ganzflächig, eine elastisch nachgiebige Auflage (Innenhaut 9), insbesondere Schaumstoff, aufweist.

4. Stütze nach Anspruch 3, dadurch gekennzeichnet, dass die Auflage eine Dicke zwischen 1,5 und 3,0 mm, vorzugsweise 2,0 mm hat.

5. Stütze nach Anspruch 1, dadurch gekennzeichnet, dass zumindestent der Stützschaft (7) durch die Aussenhaut (8) überdeckt ist.

6. Stütze nach Anspruch 1 oder 3, dadurch gekennzeichnet, dass die Aussenhaut (8) und ggf. die Auflage (9) den Stützschaft (7) randseitig überragen und in diesem Überstand miteinander verbunden, insbesondere verklebt sind.

7. Stütze nach einem der vorhergehenden Ansprüche, dadurch gekennzeichnet, dass sie als Schuheinlage ausgebildet ist.

8. Stütze nach einem der vorhergehenden Ansprüche, dadurch gekennzeichnet, dass sich die Aussenhaut (8) über den gesamten Fussbereich erstreckt und diesen einhüllt, und dass ggf. eine Laufsohle aufgebracht ist.

## Claims

1. Supporting device for the ankle-joint area comprising a supporting upper (7) at least partially enclosing both the tarsal area (4) and the heel area (5) as well as part of the lower part of the leg (6) and being essentially rigid in the supporting direction and having at the front an opening (11) closable by adjustable fastener elements preferably comprising hook and loop fasteners, characterized in that said supporting upper (7) extends, on the one hand, over approximately the tarsal bone (4) or approximately half of the length of the foot, respectively, and, on the other hand, as far as at least approximately 5 cm above the ankle-bones and in the region of the ankle-bones has recesses (16) covered by an outer skin (8).

2. Supporting device as defined in claim 1, characterized in that said supporting upper (7) also has a recess (16a) in the heel area.

3. Supporting device as defined in one of the preceding claims, characterized in that the partial shoe (3) has on its inner side, in the given circumstances, over the entire surface, an elastically resilient layer (inner skin 9), in particular foamed material.

4. Supporting device as defined in claim 3, characterized in that said layer is approximately between 1.5 and 3.0 mm, preferably 2.00 mm thick.

5. Supporting device as defined in claim 1, characterized in that at least said supporting upper (7) is covered by said outer skin (8).

6. Supporting device as defined in claim 1 or 3, characterized in that said outer skin (8) and, in the given circumstances, said layer (9) extend beyond the edges of said supporting upper (7) and are joined together, in particular adhesively, in this extension.

7. Supporting device as defined in one of the preceding claims, characterized in that it is designed as a shoe support.

8. Supporting device as defined in one of the preceding claims, characterized in that said outer skin (8) extends over the entire area of the foot and encloses it, and in that in the given circumstances an outsole is attached to it.

**Revendications**

1. Organe de soutien pour la région de l'articulation tibiotarsienne, avec un montant-tige (7) qui enclôt au moins partiellement la région du tarse (4) tout aussi bien que la région du talon (5) et qu'une partie du bas de la jambe (6) et qui est sensiblement rigide dans la direction du soutien et présente, du côté avant, une ouverture (11) pouvant être fermée au moyen d'éléments de fermeture réglables présentant de préférence des moyens de fermeture auto-agrippants, caractérisé par le fait que le montant-tige (7) s'étend d'une part sensiblement par-dessus les os du tarse (4), ou encore sur environ la moitié de la longueur du pied, et s'étend d'autre part jusqu'à au moins environ 5 cm au-dessus de la cheville et présente, dans la région de la cheville, des évidements (16) qui sont revêtus par une peau extérieure (8).

2. Organe de soutien selon revendication 1, caractérisé par le fait que le montant-tige (7) présente également un évidement (16a) dans la région du talon.

3. Organe de soutien selon l'une des revendications précédentes, caractérisé par le fait que la chaussure partielle (3) présente intérieurement, éventuellement sur toute sa surface, un garnissage (peau intérieure 9), notamment une matière-mousse, déformable élastiquement.

4. Organe de soutien selon revendication 3, caractérisé par le fait que le garnissage possède une épaisseur comprise entre 1,5 et 3,0 mm, de préférence égale à 2,0 mm.

5. Organe de soutien selon la revendication 1, caractérisé par le fait qu'au moins le montant-tige (7) est recouvert par la peau extérieure (8).

6. Organe de soutien selon revendication 1 ou 3, caractérisé par le fait que la peau extérieure (8) et éventuellement le garnissage (9) débordent au-delà du bord du montant-tige (7) et sont mutuellement assemblés, notamment par collage, dans cette partie débordante.

7. Organe de soutien selon l'une des revendications précédentes, caractérisé par le fait qu'il est réalisé en tant que semelle orthopédique.

8. Organe de soutien selon l'une des revendications précédentes, caractérisé par le fait que la peau extérieure (8) s'étend sur l'ensemble de la zone du pied qu'elle enveloppe, et par le fait qu'une semelle de marche est éventuellement rapportée.